# EUROPÄISCHE PATENTSCHRIFT

(11) **EP 1 139 988 B1**
(45) Veröffentlichungstag und Bekanntmachung des Hinweises auf die Patenterteilung: **25.09.2002**
(21) Anmeldenummer: 99963526.1
(22) Anmeldetag: 14.12.1999
(51) Int. Cl.: A61K 7/13

(54) **MITTEL ZUM FÄRBEN VON KERATINHALTIGEN FASERN**
AGENT FOR DYING KERATIN FIBERS
AGENTS PERMETTANT DE COLORER LES FIBRES DE KERATINE

(30) Priorität: 23.12.1998 DE 19859750
(43) Veröffentlichungstag der Anmeldung: 10.10.2001
(73) Patentinhaber: Henkel Kommanditgesellschaft auf Aktien, 40589 Düsseldorf-Holthausen (DE)
(72) Erfinder: MÖLLER, Hinrich, D-40789 Monheim (DE); OBERKOBUSCH, Doris, D-40591 Düsseldorf (DE); HÖFFKES, Horst, D-40595 Düsseldorf (DE)
(86) Internationale Anmeldenummer: EP9909908
(87) Internationale Veröffentlichungsnummer: WO00038633

(56) Entgegenhaltungen:
- DE-A- 2 334 738
- DE-A- 2 603 848
- HOWITZ, J. UND PHILIPP, J.: "Über Bromierung des p-Toluchinolins und über p-Chinolinaldehyd" JUSTUS LIEBIGS ANN. CHEM., Nr. 396, 1913, Seiten 23-37, XP000892342

## Beschreibung

Die Erfindung betrifft ein Mittel zum Färben von keratinhaltigen Fasern, insbesondere menschlichen Haaren, das Chinoliniumaldehyde und -ketone enthält, die Verwendung dieser Aldehyde oder Ketone als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen Chinoliniumaldehyde oder -ketone zum Färben von keratinhaltigen Fasem ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemittein mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten Chinoliniumaldehyde oder -ketone sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend Chinoliniumaldehyde oder -ketone mit der Formel I, 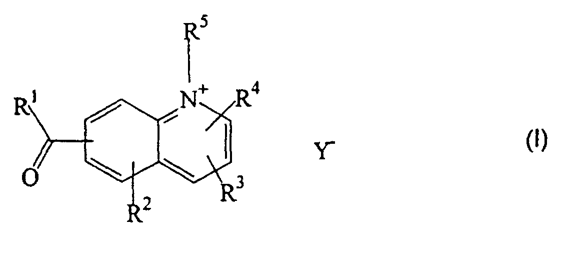 worin
R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alk(en)ylgruppe, Arylgruppe, Arylalkylgruppe oder Heteroarylgruppe steht,
Y⁻ für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern, verwendet werden.

Vorzugsweise sind die Verbindungen mit der Formel I ausgewählt aus der Gruppe folgender Chinoliniumaldehyde und -ketone: Benzolsulfonate, Tetrafluorborate, p-Toluolsulfonate, Methansulfonate, Trifluormethansulfonate, Perchlorate, Sulfate, Chloride, Bromide, Jodide, Tetrafluorborate und/oder Tetrachlorzinkate von 5-Formyl-1-methyl-chinolinium, 6-Formyl-1-methylchinolinium, 7-Formyl-1-methyl-chinolinium, 8-Formyl-1-methyl-chinolinium, 5-Formyl-1-ethyl-chinolinium, 6-Formyl-1-ethyl-chinolinium, 7-Formyl-1-ethylchinolinium, 8-Formyl-1-ethyl-chinolinium, 5-Formyl-1-benzyl-chinolinium, 6-Formyl-1-benzyl-chinolinium, 7-Formyl-1-benzyl-chinolinium, 8-Formyl-1-benzylchinolinium, 5-Formyl-1-allyl-chinolinium, 6-Formyl-1-allyl-chinolinium, 7-Formyl-1-allyl-chinolinium, 8-Formyl-1-allylchinolinium, 5-Acetyl-1-methyl-chinolinium, 6-Acetyl-1-methyl-chinolinium, 7-Acetyl-1-methyl-chinolinium, 8-Formyl-1-Acetylchinolinium sowie deren beliebigen Gemischen.

Diese Substanzen sind literaturbekannt oder im Handel erhältlich.

Die voranstehend genannten Chinoliniumaldehyde und -ketone mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente die Chinoliniumaldehyde und -ketone mit der Formel I allein enthalten, werden bevorzugt für Färbungen im Gelb-, Orange- und Braunbereich eingesetzt. Färbungen mit noch erhöhter Brillanz und verbesserten Echtheitseigenschaften (Lichtechtheit, Waschechtheit, Reibechtheit) über einen weiten Nuancenbereich von gelb über braungelb, braunorange, mittelbraun, dunkelbraun, rotbraun, rotviolett bis hin zu schwarz werden erzielt, wenn die erfindungsgemäß eingesetzten Verbindungen mit der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den aromatischen Aldehyden und Ketonen der Formel I brillante Färbungen ergeben. Andererseits sind darunter aber auch Verbindungen, die bereits als Oxidationsfarbstoffvorprodukte eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Chinoliniumaldehyde und -ketone der Formel I gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von Chinoliniumaldehyden und -ketonen der Formel I mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wäßrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 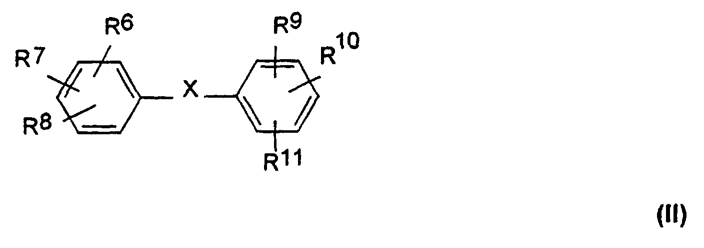 in der R⁶ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
R⁷, R⁸, R⁹, R¹⁰ und R¹¹ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und

X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

Z-(CH₂-Y-CH₂-Z')ₒ (III)

in der
- Y: eine direkte Bindung, eine CH₂- oder CHOH-Gruppe bedeutet,
- Z: und Z' unabhängig voneinander für ein Sauerstoffatom, eine NR¹²- Gruppe, worin R¹² Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
- o: eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Als Aminosäuren kommen bevorzugt alle natürlich vorkommenden und synthetischen α-Aminosäuren in Frage, z. B. die durch Hydrolyse aus pflanzlichen oder tierischen Proteinen, z. B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Omithin, Lysin und Tryptophan. Aber auch andere Aminosäuren, wie z. B. 6-Aminocapronsäure, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z. B. Glutathion und die in den Hydrolysaten von Kollagen, Keratin, Casein, Elastin, Sojaprotein, Weizengluten oder Mandelprotein enthaltenen Oligopeptide zu nennen. Bevorzugt sind dabei Mittel, die die Verbindungen mit der Formel I gemeinsam mit Verbindungen mit primärer oder sekundärer Aminogruppe oder mit aromatischen Hydroxyverbindungen enthalten.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salzen.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'carbonsäure, 6-Nitro-1,2,3,4-tetrahydrochinoxalin, 4-N-Ethyl-1,4-bis(2'hydroxyethylamino)-2-nitrobenzol-hydrochlorid und 1-Methyl-3-nitro-4-(2'hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-lmidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentpnit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Hamstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammoniumoder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von Chinoliniumaldehyden oder -ketonen mit der Formel I, 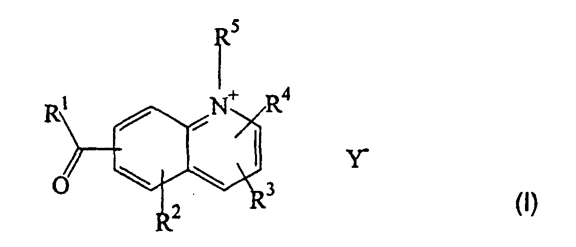 worin
- R¹: für ein Wasserstoffatom, eine (C₁-C₄)-Alkylgruppe, eine Arylgruppe oder eine Heteroarylgruppe steht,
- R², R³ und R⁴: jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
- R⁵: für eine (C₁-C₄)-Alk(en)ylgruppe, Arylgruppe, Arylalkylgruppe oder Heteroarylgruppe steht,
- Y⁻: für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht,
als eine färbende Komponente in Haarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A) mindestens einen Chinoliniumaldehyd oder ein Chinoliniumketon mit der Formel I, 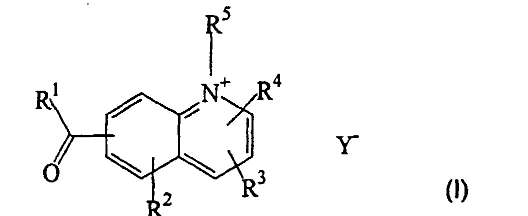
worin
R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alk(en)ylgruppe, Arylgruppe, Aralkylgruppe oder Heteroarylgruppe steht,
Y⁻ für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht.
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Chinoliniumaldehyde oder -ketone der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Chinoliniumaldehyde oder -ketone der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines Chinoliniumaldehyds oder - ketons mit der Formel I, 5 mmol eines Oxidationsfarbstoffvorproduktes der Komponente B und 5 mmol Natriumacetat und einen Tropfen einer 20-%igen Fettalkylethersulfat-Lösung in 50 ml Wasser bei 60°C hergestellt. Die Aufschlämmungen wurden bei 30°C miteinander vermischt und mit verdünnter Salzsäure oder Natraonlauge auf einen pH-Wert von 6 eingestellt.

In diese Färbelösung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| **Ausfärbungen mit 7-Formyl-1-methylchinolinium-trifluormethansulfonat** | | |
|---|---|---|
| **Komponente B** | **Färbenuance** | **Farbtiefe** |
| 2,5-Diaminotoluol x H₂SO₄ | dunkel-orangebraun | ++(+) |
| 2-Methylamino-3-amino-6-methoxypyridin x 2HCl | dunkelbraun | ++(+) |
| 2-(2,5-Diaminophenyl)-ethanol | | |
| x H₂SO₄ | orangebraun | ++ |
| 2-Aminomethyl-4-aminophenol | | |
| x 2 HCl | braungelb | ++ |
| N, N-Bis-(2-hydroxyethyl)-p-phenylendiamin x HCl | rotviolett | ++ |
| 2-Amino-4-(2-hydroxyethylamino)- | | |
| anisol x H₂SO₄ (Lehmanns Blau) | hellbraun | +(+) |
| 3,5-Diamino-2,6-dimethoxypyridin x 2HCl | schwarz | +++ |

**Tabelle 2**

| **Ausfärbungen mit 6-Formylchinolinium-trifluormethansulfonat** | | |
|---|---|---|
| **Komponente B** | **Färbenuance** | **Farbtiefe** |
| 2,5-Diaminotoluol x H₂SO₄ | braunorange | ++ |
| 2-Methylamino-3-amino-6-methoxypyridin x 2HCl | dunkelbraun | ++ |
| 2-(2,5-Diaminophenyl)-ethanol x H₂SO₄ | orangebraun | ++ |
| 2-Aminomethyl-4-aminophenol x 2 HCI | braungelb | ++ |
| N,N-Bis-(2-hydroxyethyl)-p-phenylendiamin x HCl ++(+) | rotbraun | |
| 2-Amino-4-(2-hydroxyethylamino)-anisol x H₂SO₄ (Lehmanns Blau) | hellbraun | +(+) |
| 3,5-Diamino-2,6-dimethoxypyridin x 2HCl | schwarz | +++ |

## Patentansprüche

1. Mittel zum Färben von keratinhaltigen Fasern, insbesondere menschlichen Haaren, enthaltend als färbende Komponente mindestens einen aromatischen Aldehyd oder ein aromatisches Keton mit der Formel I, 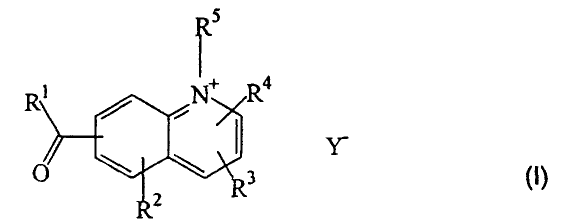 worin
n R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alk(en)ylgruppe, Arylgruppe, Arylalkylgruppe oder Heteroarylgruppe steht,
Y⁻ für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht.

2. Mittel nach Anspruch 1, **dadurch gekennzeichnet, daß** als Verbindungen mit der Formel I Benzolsulfonate, Tetrafluorborate, p-Toluolsulfonate, Methansulfonate, Trifluormethansulfonate, Perchlorate, Sulfate, Chloride, Bromide, Jodide, Tetrafluorborate und/oder Tetrachlorzinkate von 5-Formyl-1-methyl-chinolinium, 6-Formyl-1-methyl-chinolinium, 7-Formyl-1-methylchinolinium, 8-Formyl-1-methyl-chinolinium, 5-Formyl-1-ethyl-chinolinium, 6-Formyl-1-ethyl-chinolinium, 7-Formyl-1-ethyl-chinolinium, 8-Formyl-1-ethylchinolinium, 5-Formyl-1-benzyl-chinolinium, 6-Formyl-1-benzyl-chinolinium, 7-Formyl-1-benzyl-chinolinium, 8-Formyl-1-benzyl-chinolinium, 5-Formyl-1-allyl-chinolinium, 6-Formyl-1-allyl-chinolinium, 7-Formyl-1-allyl-chinolinium, 8-Formyl-1-allylchinolinium, 5-Acetyl-1-methyl-chinolinium, 6-Acetyl-1-methylchinolinium, 7-Acetyl-1-methyl-chinolinium, 8-Formyl-1-Acetyl-chinolinium sowie deren beliebigen Gemische eingesetzt werden.

3. Mittel nach einem der Ansprüche 1 bis 2, **dadurch gekennzeichnet, daß** die Chinoliniumaldehyde und -ketone mit der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** es zusätzlich mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung, enthält.

5. Mittel nach Anspruch 4, **dadurch gekennzeichnet, daß** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino), 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 4-Amino-2-hydroxymethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, - benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und
CH-aktiven Verbindungen ausgewählt aus der Gruppe, bestehend aus 1,2,3,3-Tetramethyl-3H-indoliumiodid, 1,2,3,3-Tetramethyl-3H-indolium-ptoluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

6. Mittel nach Anspruch 5, **dadurch gekennzeichnet, daß** die weitere Verbindung ausgewählt ist aus der Gruppe bestehend aus N-(2-Hydroxyethyl)-N-ethyl-, 2-Chlor-p-phenylendiamin, N,N-Bis-(2-hydroxyethyl)-pphenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

7. Mittel nach einem der Ansprüche 1 bis 6, **dadurch gekennzeichnet, daß** es direkt ziehende Farbstoffe aus der Gruppe der Nitrophenylendiamine, Nitroaminophenole, Anthrachinone oder Indophenole, vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

9. Mittel nach einem der Ansprüche 1 bis 8, **dadurch gekennzeichnet, daß** es Oxidationsmittel, insbesondere H₂O₂, in einer Menge von 0,01 bis 6 Gew.-%, bezogen auf die Anwendungslösung, enthält.

10. Mittel nach einem der Ansprüche 1 bis 9, **dadurch gekennzeichnet, daß** es anionische, zwitterionische oder nichtionische Tenside enthält.

11. Verwendung von Chinoliniumaldehyden oder -ketonen mit der Formel I, 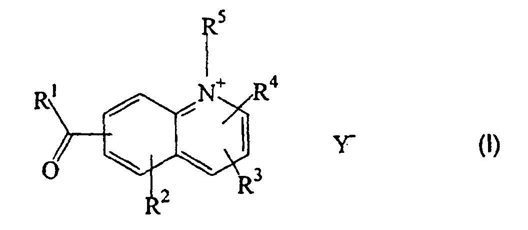 worin
R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alk(en)ylgruppe, Arylgruppe, Arylalkylgruppe oder Heteroarylgruppe steht,
Y⁻ für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht,
als eine färbende Komponente in Haarfärbemitteln.

12. Verfahren zum Färben von keratinhaltigen Fasern, insbesondere menschlichen Haaren, worin ein Färbemittel, enthaltend
A) mindestens einen Chinoliniumaldehyd oder ein Chinoliniumketon mit der Formel I, 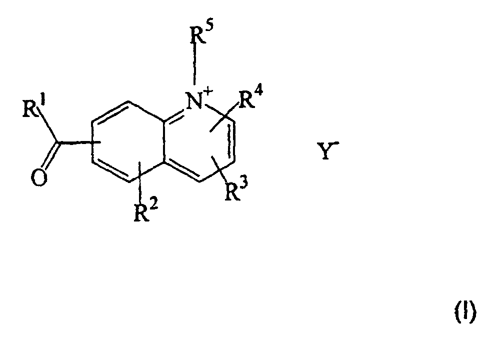 worin
n R¹ für ein Wasserstoffatom, eine (C₁-C₄)-Alkylgruppe, eine Arylgruppe oder eine Heteroarylgruppe steht,
R², R³ und R⁴ jeweils unabhängig voneinander ein Wasserstoffatom, ein Halogenatom, eine (C₁-C₄)-Alkylgruppe, eine (C₁-C₄)-Alkoxygruppe, Hydroxy-(C₁-C₄)-alkoxygruppe, Hydroxygruppe, Nitrogruppe, Aminogruppe, die durch (C₁-C₄)-Alkylgruppen substituiert sein kann, oder (C₁-C₄)-Acylgruppe bedeuten, wobei auch zwei der Reste gemeinsam einen ankondensierten Benzolring bilden können,
R⁵ für eine (C₁-C₄)-Alk(en)ylgruppe, Arylgruppe, Arylalkylgruppe oder Heteroarylgruppe steht,
Y⁻ für Halogenid, Benzolsulfonat, Tetrafluorborat, p-Toluolsulfonat, Methansulfonat, Trifluormethansulfonat, Perchlorat, Sulfat, Hydrogensulfat oder Tetrachlorzinkat oder das N-Oxid des Heterocyclus steht,
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. An agent for dyeing keratin fibers, in particular human hair, comprising, as dyeing component, at least one aromatic aldehyde or one aromatic ketone of the formula I, 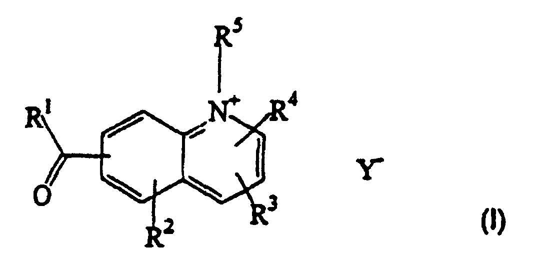 in which
R¹ is a hydrogen atom, a (C₁-C₄)-alkyl group, an aryl group or a heteroaryl group,
R², R³ and R⁴, in each case independently of one another, are a hydrogen atom, a halogen atom, a (C₁-C₄)-alkyl group, a (C₁-C₄)-alkoxy group, hydroxy-(C₁-C₄)-alkoxy group, hydroxyl group, nitro group, amino group which can be substituted by (C₁-C₄)-alkyl groups, or (C₁-C₄)-acyl group, where two of the radicals can also together form a fused benzene ring,
R⁵ is a (C₁-C₄)-alk(en)yl group, aryl group, arylalkyl group or heteroaryl group,
Y⁻ is halide, benzenesulfate, tetrafluoroborate, p-toluenesulfonate, methanesulfonate, trifluoromethanesulfonate, perchlorite, sulfate, hydrogensulfate or tetrachlorozincate or the N-oxide of the heterocycle.

2. The agent as claimed in claim 1, **characterized in that** benzenesulfonates, tetrafluoroborates, p-toluenesulfonates, methanesulfonates, trifluoromethanesulfonates, perchlorates, sulfates, chlorides, bromides, iodides, tetrafluoroborates and/or tetrachlorozincates of 5-formyl-1-methylquinolinium, 6-formyl-1-methylquinolinium, 7-formyl-1-methylquinolinium, 8-formyl-1-methylquinolinium, 5-formyl-1-ethylquinolinium, 6-formyl-1-ethylquinolinium, 7-formyl-1-ethylquinolinium, 8-formyl-1-ethylquinolinium, 5-formyl-1-benzylquinolinium, 6-formyl-1-benzylquinolinium, 7-formyl-1-benzylquinolinium, 8-formyl-1-benzylquinolinium, 5-formyl-1-allylquinolinium, 6-formyl-1-allylquinolinium, 7-formyl-1-allylquinolinium, 8-formyl-1-allylquinolinium, 5-acetyl-1-methylquinolinium, 6-acetyl-1-methylquinolinium, 7-acetyl-1-methylquinolinium, 8-formyl-1-acetylquinolinium
and any mixtures thereof are used as compounds of the formula I.

3. The agent as claimed in any of claims 1 to 2, **characterized in that** quinolinium aldehydes and ketones the of the formula I are present in an amount of from 0.03 to 65 mmol, in particular from 1 to 40 mmol, based on 100 g of the total dyeing agent.

4. The agent as claimed in any of claims 1 to 3, **characterized in that** it additionally comprises at least one compound having a primary or secondary amino group or hydroxyl group, chosen from primary or secondary aliphatic or aromatic amines, nitrogen-containing heterocyclic compounds, amino acids, oligopeptides constructed from 2 to 9 amino acids, and aromatic hydroxy compounds, and/or at least one CH-active compound.

5. The agent as claimed in claim 4, **characterized in that** the further compound is chosen from primary or secondary amines from the group consisting of N- (2-hydroxyethyl)-N-ethyl-, N-(2-methoxyethyl)-, 2,3-, 2,4-, 2,5-dichloro-p-phenylenediamine, 2-chloro-p-phenylenediamine, N,N-bis(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro, 6-methyl-3-amino-2-chloro, 5-(2-hydroxyethylamino)-4-methoxy-2-methyl-, 4-amino-2-aminomethylphenol, 4-amino-2-hydroxymethylphenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalenesulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic nitriles, anilines, in particular nitro group-containing anilines, such as 4-nitroaniline, 4-nitro-1,3-phenylenediamine, 2-nitro-4-amino-1-(2-hydroxyethylamino)benzene, 2-nitro-1-amino-4-[bis(2-hydroxyethyl)amino]benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, aromatic anilines and phenols containing a further aromatic radical, such as 4,4'-diaminostilbene dihydrochloride, 4,4'-diaminostilbene-2,2'-disulfonic acid, Na salt, 4,4'-diaminodiphenylmethane, sulfide, sulfoxide, -amine, 4,4'-diaminodiphenylamine-2-sulfonic acid, 4,4'-diaminobenzophenone, -diphenyl ether, 3,3',4,4'-tetraaminodiphenyl tetrahydrochloride, 3,3',4,4'-tetraaminobenzophenone, 1,3-bis(2,4-diaminophenoxy)propane tetrahydrochloride, 1,8-bis(2,5-diaminophenoxy)-3,6-dioxaoctane tetrahydrochloride, 1,3-bis(4-aminophenylamino)propane, -2-propanol, 1,3-bis-[N-(4-aminophenyl)-2-hydroxyethylamino] -2-propanol, N,N-bis[2-(4-aminophenoxy)ethyl]methylamine trihydrochloride,
nitrogen-containing heterocyclic compounds chosen from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5, 6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-,3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline, and indole and indoline derivatives, and 4-, 5-, 6-, 7-aminoindole, 4-, 5-, 6-, 7-hydroxyindole, 5,6-dihydroxyindole, 5,6-dihydroxyindoline and 4-hydroxyindoline, and in each case from the physiologically compatible salts of these compounds formed with preferably inorganic salts, aromatic hydroxy compounds chosen from the group consisting of 2-, 4-, 5-methylresorcinol, 2,5-dimethylresorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2-hydroxyethyl)-, 3,4-methylenedioxyphenol. 2,4-, 3,4-dihydroxy-benzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulfonic acid, 3,6-dihydroxy-2,7-naphthalenesulfonic acid and
CH-active compounds chosen from the group consisting 1,2,3,3-tetramethyl-3H-indolium iodide, 1,2,3,3-tetramethyl-3H-indolium p-toluenesulfonate, 1,2,3,3-tetramethyl-3H-indolium methanesulfonate, Fischer's base (1,3,3-trimethyl-2-methyleneindoline), 2,3-dimethylbenzothiozolium iodide, 2,3-dimethylbenzothiazolium p-toluenesulfonate, rhodanine, rhodanine-3-acetic acid, 1-methyl-2-quinaldinium iodide, 1-ethyl-2-quinaldinium iodide, 1,4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxyl acetate, cumaranone and 1-methyl-3-phenyl-2-pyrazolinone.

6. The agent as claimed in claim 5, **characterized in that** the further compound is chosen from the group consisting of N-(2-hydroxyethyl)-N-ethyl-, 2-chloro-p-phenylenediamine, N,N-bis(2-hydroxyethyl)-p-phenylenediamine, 4-aminophenol, p-phenylenediamine, 2-(2,5-diaminophenyl)ethanol, 2,5-diaminotoluene, 3,4-methylenedioxyaniline, 2-amino-4- (2-hydroxyethylamino)anisole, 2-(2,4-diaminophenoxy)ethanol, 3-amino-2,4-dichloro-, 2-methyl-5-amino-, 3-methyl-4-amino-, 2-methyl-5-(2-hydroxyethylamino) -, 2-methyl-5-amino-4-chloro-, 6-methyl-3-amino-2-chloro-, 2-aminomethyl-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5,6-dihydroxyindole and 5,6-dihydroxyindoline and in each case from the physiologically compatible salts of these compounds formed preferably with inorganic acids.

7. The agent as claimed in any of claims 1 to 6, **characterized in that** it comprises substantive dyes from the group of nitrophenylenediamines, nitroaminophenols, anthraquinones or indophenols, preferably in an amount of from 0.01 to 20% by weight, based on the total dyeing agent.

8. The agent as claimed in any of claims 1 to 7, **characterized in that** ammonium or metal salts chosen from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminum, manganese, iron, cobalt, copper or zinc are added.

9. The agent as claimed in any of claims 1 to 8, **characterized in that** it comprises oxidizing agents, in particular H₂O₂, in an amount of from 0.01 to 6% by weight, based on the use solution.

10. The agent as claimed in any of claims 1 to 9, **characterized in that** it comprises anionic, zwitterionic or nonionic surfactants.

11. The use of quinolinium aldehydes or ketones of the formula I, 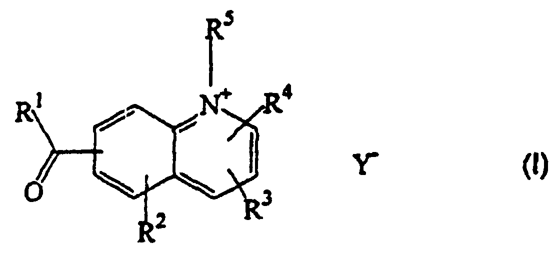 in which
R¹ is a hydrogen atom, a (C₁-C₄)-alkyl group, an aryl group or a heteroaryl group,
R², R³ and R⁴, in each case independently of one another, are a hydrogen atom, a halogen atom, a (C₁-C₄)-alkyl group, a (C₁-C₄)-alkoxy group, hydroxy-(C₁-C₄)-alkoxy group, hydroxyl group, nitro group, amino group which can be substituted by (C₁-C₄)-alkyl groups, or (C₁-C₄)-acyl group, where two of the radicals can also together form a fused benzene ring,
R⁵ is a (C₁-C₄)-alk(en)yl group, aryl group, arylalkyl group or heteroaryl group,
Y⁻ is halide, benzenesulfate, tetrafluoroborate, p-toluenesulfonate, methanesulfonate,
trifluoromethanesulfonate, perchlorite, sulfate, hydrogensulfate or tetrachlorozincate or the N-oxide of the heterocycle,
as a dyeing component in hair dyeing agents.

12. A method of dyeing keratin fibers, in particular human hair, in which a dyeing agent comprising
A) at least one quinolinium aldehyde or a quinolinium ketone of the formula I, 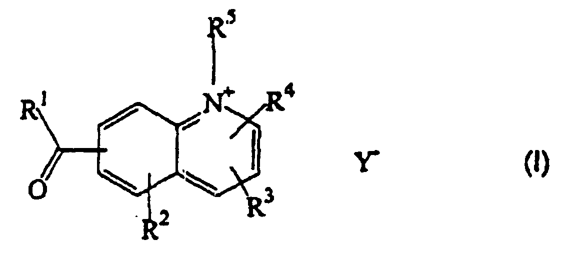 in which
which R¹ is a hydrogen atom, a (C₁-C₄)-alkyl group, an aryl group or a heteroaryl group,
R², R³ and R⁴, in each case independently of one another, are a hydrogen atom, a halogen atom, a (C₁-C₄)-alkyl group, a (C₁-C₄)-alkoxy group, hydroxy-(C₁-C₄)-alkoxy group, hydroxyl group, nitro group, amino group which can be substituted by (C₁-C₄)-alkyl groups, or (C₁-C₄)-acyl group, where two of the radicals can also together form a fused benzene ring,
R⁵ is a (C₁-C₄)-alk(en)yl group, aryl group, arylalkyl group or heteroaryl group,
Y⁻ is halide, benzenesulfate, tetrafluoroborate, p-toluenesulfonate, methanesulfonate, trifluoromethanesulfonate, perchlorite, sulfate, hydrogensulfate or tetrachlorozincate or the N-oxide of the heterocycle,
B) at least one compound having a primary or secondary amino group or hydroxyl group, chosen from primary or secondary aliphatic or aromatic amines, nitrogen-containing heterocyclic compounds, amino acids, oligopeptides constructed from 2 to 9 amino acids, and aromatic hydroxy compounds, and/or at least one CH-active compound,
and customary cosmetic ingredients, is applied to the keratin fibers, left on the fibers for a while, usually about 30 minutes, and then rinsed out again or washed out using a shampoo.

## Revendications

1. Agent de coloration des fibres contenant de la kératine, en particulier des cheveux humains, contenant comme composant colorant au moins un aldéhyde aromatique ou une cétone aromatique de formule I, 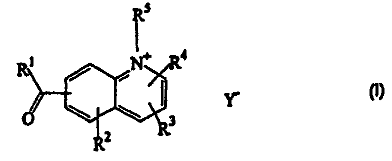 dans laquelle R¹ représente un atome d'hydrogène, un groupe alkyle en C₁ à C₄, un groupe aryle ou un groupe hétéroaryle,
R², R³ et R⁴ représentent chacun indépendamment l'un de l'autre un atome d'hydrogène, un atome d'halogène, un groupe alkyle en C₁ à C₄, un groupe alcoxy en C₁ à C₄, un groupe hydroxy-alcoxy en C₁ à C₄, un groupe hydroxy, un groupe nitro, un groupe amino, qui peut être substitué par des groupes allyle en C₁ à C₄, ou un groupe acyle en C₁ à C₄, deux des radicaux pouvant également former ensemble un noyau benzène condensé,
R⁵ représente un groupe alc(én)yle en C₁ à C₄, un groupe aryle, un groupe arylalkyle, ou un groupe hétéroaryle,
Y- représente un halogénure, un benzène sulfonate, un tétrafluoroborate, un p-toluène sulfonate, un méthane sulfonate, un trifluorométhane sulfonate, un perchlorate, un sulfate, un sulfate acide ou un tétrachlorozincate, ou le N-oxyde de l'hétérocycle.

2. Agent selon la revendication 1,
**caractérisé en ce qu'**
on utilise comme composés de formule I des benzènes sulfonates, tétrafluoroborates, p-toluène sulfonates, méthane sulfonates, trifluorométhane sulfonates, perchlorates, sulfates, chlorures, bromures, iodures, tétrafluoroborates et/ou tétrachlorozincates de 5-formyl-1-méthylquinolinium, 6-formyl-1-méthyl-quinolinium, 7-formyl-1-méthyl-quinolinium, 8-formyl-1-méthyl-quinolinium, 5-formyl-1-éthyl-quinolinium, 6-formyl-1-éthyl-quinolinium, 7-formyl-1-éthyl-quinolinium, 8-formyl-1-éthyl-quinolinium, 5-formyl-1-benzyl-quinolinium, 6-formyl-1-benzylquinolinium, 7-formyl-1-benzyl-quinolinium, 8-formyl-1-benzyl-quinolinium, 5-formyl-1-allyl-quinolinium, 6-formyl-1-allyl-quinolinium, 7-formyl-1-allyl-quinolinium, 8-formyl-1-allylquinolinium, 5-acétyl-1-méthyl-quinolinium, 6-acétyl-1-méthyl-quinolinium, 7-acétyl-1-méthylquinolinium, 8-formyl-1-acétyl-quinolinium ainsi que leurs mélanges quelconques.

3. Agent selon l'une des revendications 1 à 2,
**caractérisé en ce que**
les aldéhydes et cétones de quinolinium de formule I sont contenus en une quantité de 0,03 à 65 mmole, en particulier de 1 à 40 mmole, pour 100 g de l'ensemble du colorant.

4. Agent selon l'une des revendications 1 à 3,
**caractérisé en ce qu'**
il contient en outre au moins un composé à groupe amino primaire ou secondaire ou à groupe hydroxy choisi parmi les amines aliphatiques ou aromatiques primaires ou secondaires, les composés hétérocycliques contenant de l'azote, les acides aminés, les oligopeptides constitués de 2 à 9 acides aminés et les composés hydroxy aromatiques, et/ou au moins un composé CH-actif.

5. Agent selon la revendication 4,
**caractérisé en ce que**
l'autre composé est choisi parmi les amines primaires ou secondaires du groupe constitué par la N-(2-hydroxyéthyl)-N-éthyl-, N-(2-méthoxyéthyl-), 2,3-, 2,4-, 2,5-dichloro-p-phénylène diamine, 2-chloro-p-phénylène diamine, N,N-bis-(2-hydroxyéthyl)-p-phénylène diamine, 2,5-dihydroxy-4-morpholinoaniline-dihydrobromure, 2-, 3-, 4-aminophénol, o-, m-, p-phénylène diamine, 2,4-diaminophénoxyéthanol, 2-(2,5-diaminophényl)-éthanol, 2,5-diaminotoluène, -phénol, -phénéthol, 4-méthylamino-, 3-amino-4-(2'-hydroxyéthyloxy)-, 3,4-méthylènediamino-, 3,4-méthylène dioxyaniline, 3-amino-2,4-dichloro-, 4-méthylamino-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 5-(2-hydroxyéthylamino)-4-méthoxy-2-méthyl-, 4-amino-2-aminométhylphénol, 4-amino-2-hydroxyméthyl-phénol, 1,3-diamino-2,4-diméthoxybenzène, acide 2-, 3-, 4-aminobenzoïque, acide phénylacétique, acide 2,3-, 2,4-, 2,5-, 3,4-, 3,5-diaminobenzoïque, acide 4-, 5-aminosalicylique, acide 3-amino-4-hydroxy-, 4-amino-3-hydroxybenzoïque, acide 2-, 3-, 4-aminobenzène sulfonique, acide 3-amino-4-hydroxybenzène sulfonique, acide 4-amino-3-hydroxynaphtalènesulfonique, acide 6-amino-7-hydroxynaphtalène-2-sulfonique, acide 7-amino-4-hydroxynaphtalène-2-sulfonique, acide 4-amino-5-hydroxynaphtalène-2,7-disulfonique, acide 3-amino-2-naphtoïque, acide 3-aminophtalique, acide 5-aminoisophtalique, 1,3,5-, 1,2,4-triaminobenzène, tétrachlorhydrate de 1,2,4,5-tétraaminobenzène, trichlorhydrate de 2,4,5-triaminophénol, pentachlorhydrate de pentaaminobenzène, héxachlorhydrate d'hexaaminobenzène, trichlorhydrate de 2,4,6-triaminorésorcine, sulfate de 4,5-diaminopyrocatéchine, dichlorhydrate de 4,6-diaminopyrogallol, sulfate de 3,5-diamino-4-hydroxypyrocatéchine, nitriles aromatiques, anilines, en particulier anilines contenant des groupes nitro, comme 4-nitroaniline, 4-nitro-1,3-phénylène diamine, 2-nitro-4-amine-1-(2-hydroxyéthylamino)-benzène, 2-nitro-1-amino-4-[bis-(2-hydroxyéthyl)-amino]-benzène, acide 4-amino-2-nitrodiphénylamine-2'-carboxylique, 2-amino-6-chloro-4-nitrophénol, 1-amino-5-chloro-4-(2-hydroxyéthylamino)-2-nitrobenzène, anilines aromatiques, ou selon les cas phénols ayant un autre radical aromatique comme le dichlorhydrate de 4,4'-diaminostilbène, le sel de sodium de l'acide 4,4'-diaminostilbène-2,2'-disulfonique, le 4,4'-diaminodiphénylméthane, -sulfure, -sulfoxyde, -amine, l'acide 4,4'-diaminodiphénylamine-2-sulfonique, la 4,4'-diaminobenzophénone, -diphényléther, le tétrachlorhydrate de 3,3',4,4'-tétraaminodiphényle, la 3,3',4,4'-tétraamino-benzophénone, le tétrachlorhydrate de 1,3-bis-(2,4-diaminophénoxy)-propane, le tétrachlorhydrate de 1,8-bis-(2,5-diaminophénoxy)-3,6-dioxaoctane, le 1,3-bis-(4-aminophénylamino)propane, -2-propanol, le 1,3-bis-[N-(4-aminophényl)-2-hydroxyéthylamino]-2-propanol, le trichlorhydrate de N,N-bis-[2-(4-aminophénoxy)-éthyl]-méthylamine,
les composés hétérocycliques azotés choisis dans le groupe constitué par la 2-, 3-, 4-amino-, 2-amino-3-hydroxy-, 2,6-diamino-, 2,5-diamino-, 2,3-diamino-, 2-diméthylamino-5-amino-, 3-amino-2-méthylamino-6-méthoxy-, 2,3-diamino-6-méthoxy-, 3,5-diamino-2,6-diméthoxy-, 2,4,5-triamino-, 2,6-dihydroxy-3,4-diméthylpyridine, 4,5,6-triamino-,2-hydroxy-4,5,6-triamino, 4-hydroxy-2,5,6-triamino-, 2,4,5,6-tétraamino-, 2-méthylamino-4,5,6-triamino-, 2,4-, 4,5-diamino-, 2-amino-4-méthoxy-6-méthyl-pyrimidine, 2,3,4-triméthylpyrrole, 2,4-diméthyl-3-éthyl-pyrrole, 3,5-diaminopyrazole, -1,2,4-triazole, 3-amino-, 3-amino-5-hydroxypyrazole, 2-, 3-, 8-aminoquinoléine, 4-aminoquinaldine, acide 2-, 6-aminonicotinique, 5-aminoisoquinoléine, 5-, 6-aminoindazole, 5-, 7-aminobenzimidazole, -benzothiazole, 2,5-dihydroxy-4-morpholinoaniline ainsi que des dérivés d'indole et d'indoline, comme le 4-, 5-, 6-, 7-aminoindole, le 4-, 5-, 6-, 7-hydroxyindole, le 5,6-dihydroxyindole, la 5,6-dihydroxyindoline et la 4-hydroxyindoline, ainsi que les sels physiologiquement acceptables de ces composés formés de préférence à partir d'acides inorganiques,
les composés hydroxy aromatiques choisis dans le groupe constitué par la 2-, 4-, 5-méthylrésorcine, la 2,5-diméthylrésorcine, la résorcine, le 3-méthoxyphénol, la pyrocatéchine, l'hydroquinone, le pyrogallol, la phloroglucine, l'hydroxyhydroquinone, le 2-, 3-, 4-méthoxy-, 3-diméthylamino-, 2-(2-hydroxyéthyl)-, 3,4-méthylènedioxyphénol, l'acide 2,4-, 3,4-dihydroxybenzoïque, l'acide phénylacétique, l'acide gallique, l'acide 2,4,6-trihydroxybenzoïque, l'acétophénone, la 2-, 4-chlororésorcine, le 1-naphtol, le 1,5-, 2,3-, 2,7-dihydroxynaphtalène, l'acide 6-diméthylamino-4-hydroxy-2-naphtalène sulfonique, l'acide 3,6-dihydroxy-2,7-naphtalène sulfonique, et
les composés CH-actifs choisis dans le groupe constitué par l'iodure de 1,2,3,3-tétraméthyl-3H-indolium, le p-toluène sulfonate de 1,2,3,3-tétraméthyl-3H-indolium, le méthane sulfonate de 1,2,3,3-tétraméthyl-3H-indolium, les bases de Fischer (1,3,3-triméthyl-2-méthylèneindoline), l'iodure de 2,3-diméthylbenzothiazolium, le p-toluène sulfonate de 2,3-diméthyl-benzothiazolium, la rhodanine, l'acide rhodanine-3-acétique, l'iodure de 1-méthyl-2-quinalidinium, l'iodure de 1-éthyl-2-quinaldinium, l'iodure de 1,4-diméthylquinolinium, l'acide barbiturique, l'acide thiobarbiturique, l'acide 1,3-diméthylthio-bartiturique, l'acide 1,3-diéthylthiobarbiturique, l'acide diéthylthiobarbiturique, l'oxindole, l'acétate de 3-indoxyle, la coumaranone et la 1-méthyl-3-phényl-2-pyrazolinone.

6. Agent selon la revendication 5,
**caractérisé en ce que**
l'autre composé est choisi dans le groupe constitué par la N-(2-hydroxyéthyl)-N-éthyl-, 2-chloro-p-phénylène diamine, la N,N-bis(2-hydroxyéthyl)-p-phénylène diamine, le 4-aminophénol, la p-phénylène diamine, le 2-(2,5-diaminophényl)éthanol, le 2,5-diaminotoluène, la 3,4-méthylène dioxyaniline, le 2-amino-4-(2-hydroxyéthylamino)anisol, le 2-(2,4-diaminophénoxy)-éthanol, le 3-amino-2,4-dichloro-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 2-aminométhyl-4-aminophénol, le 2-hydroxyméthyl-4-aminophénol, le 3,4-méthylène dioxyphénol, l'acide 3,4-diaminobenzoïque, la 2,5-diamino-, 2-diméthylamino-5-amino-, 3-amino-2-méthylamino-6-méthoxy-, 2,3-diamino-6-méthoxy-, 3,5-diamino-2,6-diméthoxy-, 2,6-dihydroxy-3,4-diméthylpyridine, la 2-hydroxy-4,5,6-triamino-, 4-hydroxy-2,5,6-triamino-, 2,4,5,6-tétraamino-, 2-méthylamino-4,5,6-triaminopyrimidine, le 3,5-diaminopyrazole, le 3-amino-5-hydroxypyrazole, le 5,6-dihydroxyindole, et la 5,6-dihydroxyindoline, ainsi que leurs sels physiologiquement acceptables formés de préférence avec des acides inorganiques.

7. Agent selon l'une des revendications 1 à 6,
**caractérisé en ce qu'**
il contient des colorants directs du groupe des nitrophénylène diamines, nitroaminophénols, anthraquinones ou indophénols, de préférence en une quantité de 0,01 à 20 % en poids par rapport à l'ensemble du colorant.

8. Agent selon l'une des revendications 1 à 7,
**caractérisé en ce qu'**
on ajoute des sels d'ammonium ou de métaux choisis dans le groupe des formiates, carbonates, halogénures, sulfates, butyrates, valériates, capronates, acétates, lactates, glycolates, tartrates, citrates, gluconates, propionates, phosphates et phosphonates de métaux alcalins, comme le potassium, le sodium ou le lithium, de métaux alcalino-terreux comme le magnésium, le calcium, le strontium ou le baryum, ou d'aluminium, de manganèse, de fer, de cobalt, de cuivre ou de zinc.

9. Agent selon l'une des revendications 1 à 8,
**caractérisé en ce qu'**
il contient un oxydant, en particulier H₂O₂, en une quantité allant de 0,01 à 6 % en poids, par rapport à la solution d'application.

10. Agent selon l'une des revendications 1 à 9,
**caractérisé en ce qu'**
il contient des agents tensioactifs, anioniques hermaphrodites ou non-ioniques.

11. Utilisation d'aldéhydes ou de cétones de quinolinium de formule I, 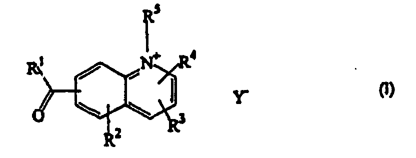 dans laquelle R¹ représente un atome d'hydrogène, un groupe alkyle en C₁ à C₄, un groupe aryle ou un groupe hétéroaryle,
R², R³ et R⁴ représentent chacun indépendamment l'un de l'autre un atome d'hydrogène, un atome d'halogène, un groupe alkyle en C₁ à C₄, un groupe alcoxy en C₁ à C₄, un groupe hydroxy-alcoxy en C₁ à C₄, un groupe hydroxy, un groupe nitro, un groupe amino, qui peut être substitué par des groupes alkyle en C₁ à C₄, ou un groupe acyle en C₁ à C₄, deux des radicaux pouvant former ensemble un noyau benzène condensé,
R⁵ représente un groupe alc(én)yle en C₁ à C₄, un groupe aryle, un groupe arylalkyle, ou un groupe hétéroaryle,
Y⁻ représente un halogénure, un benzène sulfonate, un tétrafluoroborate, un p-toluène sulfonate, un méthane sulfonate, un trifluorométhane sulfonate, un perchlorate, un sulfate, un sulfate acide ou un tétrachlorozincate, ou le N-oxyde de l'hétérocycle,
comme composant colorant dans les agents colorants pour cheveux.

12. Procédé de coloration de fibres contenant de la kératine, en particulier de cheveux humains, dans lequel on dépose sur les fibres de kératine un colorant contenant
A. au moins un aldéhyde de quinolinium ou une cétone de quinolinium de formule I, 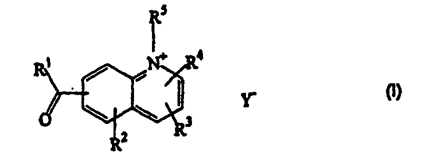 dans laquelle R¹ représente un atome d'hydrogène, un groupe alkyle en C₁ à C₄, un groupe aryle ou un groupe hétéroaryle,
R², R³ et R⁴ représentent chacun indépendamment l'un de l'autre un atome d'hydrogène, un atome d'halogène, un groupe alkyle en C₁ à C₄, un groupe alcoxy en C₁ à C₄, un groupe hydroxy-alcoxy en C₁ à C₄, un groupe hydroxy, un groupe nitro, un groupe amino, qui peut être substitué par des groupes alkyle en C₁ à C₄, ou un groupe acyle en C₁ à C₄, deux des radicaux pouvant former ensemble un noyau benzène condensé,
R⁵ représente un groupe alc(én)yle en C₁ à C₄, un groupe aryle, un groupe arylalkyle, ou un groupe hétéroaryle,
Y⁻ représente un halogénure, un benzène sulfonate, un tétrafluoroborate, un p-toluène sulfonate, un méthane sulfonate, un trifluorométhane sulfonate, un perchlorate, un sulfate, un sulfate acide ou un tétrachlorozincate, ou le N-oxyde de l'hétérocycle,
B. au moins un composé à groupe amine primaire ou secondaire ou à groupe hydroxy, choisi parmi les amines aliphatiques ou aromatiques primaires ou secondaires, les composés hétérocycliques contenant de l'azote, les acides aminés, les oligopeptides contenant de 2 à 9 acides aminés et les composés hydroxyaromatiques, et/ou au moins un composé CH-actif,
ainsi que des composants cosmétiques habituels, sur les fibres contenant de la kératine, on laisse reposer pendant quelques temps, habituellement environ 30 minutes, sur les fibres, puis on les rince à nouveau et on les lave avec un shampooing.
